# FASCICULE DE BREVET EUROPEEN

(11) **EP 3 277 224 B1**
(45) Date de publication et mention de la délivrance du brevet: **03.06.2020**
(21) Numéro de dépôt: 16711839.7
(22) Date de dépôt: 25.03.2016
(51) Int. Cl.: A61F 2/28, A61F 2/46, A61B 17/68, A61B 17/88

(54) **GABARIT DE REMODELAGE DU BANDEAU ORBITO-NASO-FRONTAL**
SCHABLONE ZUR REMODELLIERUNG DES ORBITONASOFRONTALEN BANDEAUS
ORBITO-NASO-FRONTAL BANDEAU REMODELLING TEMPLATE

(30) Priorité: 30.03.2015 FR 1552648
(43) Date de publication de la demande: 07.02.2018
(73) Titulaire: Centre Hospitalier Régional Universitaire de Besançon, 25000 Besançon (FR)
(72) Inventeur: WEBER, Elise, 25000 Besançon (FR)
(74) Mandataire: Nony
(86) Numéro de dépôt international: PCT/EP2016/056715
(87) Numéro de publication internationale: WO 2016/156270

(56) Documents cités:
- WO-A1-2014/072082
- CN-A- 103 750 907
- US-A1- 2013 006 250

## Description

La présente invention concerne le domaine de la chirurgie des déformations crâniennes, en particulier celle des sutures antérieures crâniennes encore appelée chirurgie des craniosténoses antérieures.

Les craniosténoses sont des malformations crânio-faciales fréquentes. Elles sont isolées ou intégrées dans un syndrome poly-malformatif et concernent une ou plusieurs sutures. L'atteinte des sutures antérieures est responsable de déformations orbito-frontales. Le traitement des crâniosténoses est chirurgical et son but est principalement fonctionnel, mais aussi esthétique pour la correction des déformations orbito-frontales.

La chirurgie actuelle des crâniosténoses antérieures se base sur la correction des deux parties du front : le front supérieur et le rebord sus-orbitaire, afin de restituer une forme anatomique normale au front. Un bandeau orbito-naso-frontal, selon la terminologie de Marchac et Rénier, d'environ 15 mm de haut, est réalisé pour séparer le rebord sus-orbitaire du front supérieur. Le bandeau orbito-naso-frontal est ensuite modelé de façon spécifique, en fonction de la pathologie, pour corriger les déformations sus-orbitaires. Puis le front supérieur est lui aussi remodelé.

Cette chirurgie a lieu dans la première année de vie de l'enfant, le plus souvent entre six et douze mois en l'absence de retentissement fonctionnel, afin d'utiliser la malléabilité de l'os, de permettre une réossification des pertes de substances osseuses et de corriger les déformations morphologiques.

Différentes techniques basées sur ces principes de correction du front en deux parties, avec des gestes de remodelage de la partie supérieure du front et du bandeau orbito-naso-frontal, ont été décrites et sont actuellement utilisées par plusieurs équipes. Dans ces techniques chirurgicales, le remodelage du bandeau orbito-naso-frontal est difficile à adapter de manière personnalisée à chaque enfant et difficile à apprécier en per opératoire.

On connaît par WO 2014/072082 un système pour remodeler les parties d'un crâne prélevées sur un enfant. Ce système comporte un modèle de crâne adapté à des dimensions précises de crâne, choisi par le chirurgien parmi plusieurs. Le modèle de crâne est prévu pour être en contact avec une surface intérieure desdites parties du crâne pendant l'opération chirurgicale. Un système de maintien des parties du crâne, comportant des étaux et des vis, est prévu pour être rapporté et ajusté pendant l'opération chirurgicale afin de maintenir et fixer la surface extérieure de ces parties du crâne contre le modèle de crâne. Ce système nécessite un jeu de modèles de crânes, de tailles et de formes différentes pour s'adapter au patient. De plus, le choix du modèle de crâne dépend de la subjectivité du chirurgien.

Il existe ainsi un besoin pour permettre au chirurgien d'apprécier le remodelage du bandeau orbito-naso-frontal à effectuer en per opératoire.

Il existe également un besoin pour permettre de personnaliser le remodelage du bandeau orbito-naso-frontal pour chaque enfant opéré.

La présente invention répond à tout ou partie de ces besoins en proposant un gabarit chirurgical de remodelage de bandeau orbito-naso-frontal, comportant :
- un support, et
- au moins trois surfaces d'appui destinées à être mises en contact avec un bandeau orbito-naso-frontal réalisé sur un patient de manière à en remodeler la forme en per opératoire, le positionnement des surfaces d'appui relativement au support étant fonction de la taille du crâne, définie de préférence par la distance entre les sutures fronto-zygomatiques DFZ dudit patient, mesurée dans un plan scannographique donné, en reconstruction coronale dans le plan du sommet de la crista galli, selon une mesure reproductible.

Par «DFZ », Distance entre les sutures Front-Zygomatiques, on entend la distance mesurée entre les sutures fronto-zygomatiques dans le plan coronal scannographique où est visualisé le sommet de la crista galli. La mesure de la distance entre les sutures fronto-zygomatiques est reproductible. On pourra utiliser d'autres mesures de la taille du crâne sans sortir du cadre de l'invention, par exemple en prenant la distance entre les faces internes des parois latérales des orbites

Selon un aspect particulier de l'invention, les surfaces d'appui sont définies par des éléments en saillie relativement au support, au moins un élément en saillie étant un élément rapporté sur le support, au moins un élément rapporté présentant une longueur ajustable et/ou la distance entre des éléments en saillie ou rapportés étant modifiable.

Selon un autre aspect particulier de l'invention, indépendamment ou en combinaison avec les autres aspects susmentionné et sous-mentionné, au moins une surface d'appui est plane.

Selon un autre aspect particulier de l'invention, indépendamment ou en combinaison avec les autres aspects susmentionnés, le nombre de surfaces d'appui est égal à trois ou cinq.

Au moins trois surfaces d'appui sont nécessaires de manière à définir un angle.

Grâce à l'invention, on dispose d'un outil permettant au chirurgien en per opératoire de remodeler le bandeau orbito-naso-frontal de manière adaptée, personnalisée au patient. En effet, la distance entre les sutures fronto-zygomatiques est aisément mesurable en préopératoire, à l'aide d'un scanner. De plus, cette distance est mesurée sous le bandeau orbito-naso-frontal et est donc accessible lors de l'opération chirurgicale.

Le gabarit est destiné à être mis en contact avec une surface extérieure du bandeau orbito-naso-frontal, tournée vers le front, et non avec la surface intérieure de celui-ci tournée vers le cerveau.

Aucune partie du bandeau orbito-naso-frontal n'est retirée lors du remodelage à l'aide du gabarit selon l'invention, le gabarit ne servant qu'à déformer le bandeau orbito-naso-frontal en vue de lui donner la forme souhaitée.

Avantageusement, les surfaces d'appui sont définies par des éléments en saillie relativement au support. Au moins un élément en saillie peut être rapporté sur le support. Les surfaces d'appui sont avantageusement en saillie de manière à ce que les parties du bandeau orbito-naso-frontal entre les appuis ne perturbent pas ces derniers.

Au moins un élément rapporté peut être configuré de telle sorte que le positionnement de la surface d'appui qu'il définit peut être modulé entre au moins une première position et au moins une deuxième position, différente de la première position.

En particulier, au moins un élément rapporté peut présenter une longueur ajustable de manière à permettre à la surface d'appui qu'il définit d'occuper lesdites au moins deux positions. Ledit au moins un élément rapporté présente de préférence une longueur ajustable entre deux longueurs extrémales, à savoir une longueur maximale et une longueur minimale, correspondant à des positions extrémales de la surface d'appui correspondante, et une pluralité de longueurs intermédiaires, correspondant à autant de positions intermédiaires de la surface d'appui correspondante.

Grâce à cette modulation, le même gabarit peut être ajusté pour convenir pour des patients différents, notamment des patients présentant des distances entre les sutures fronto-zygomatiques différentes.

La première position de la surface d'appui est par exemple déterminée à partir d'une reconstitution en trois dimensions d'un bandeau orbito-naso-frontal de forme idéale ayant une distance entre les sutures fronto-zygomatiques qui est prédéterminée, notamment comprise entre 60 et 90 mm.

Au moins une deuxième position de la surface d'appui est par exemple déterminée à partir d'une reconstitution en trois dimensions d'un bandeau orbito-naso-frontal de forme idéale ayant une distance entre les sutures fronto-zygomatiques qui est prédéterminée, étant notamment comprise entre 60 et 90 mm.

Les surfaces d'appui du gabarit peuvent être planes.

Lesdits au moins trois éléments rapportés peuvent comporter un élément rapporté central définissant une surface d'appui centrale et au moins deux éléments rapportés latéraux, de part et d'autre de l'élément rapporté central, définissant des surfaces d'appui latérales.

L'élément rapporté central peut être situé sur le support de manière à ce que la surface d'appui centrale se trouve sensiblement au niveau de la suture fronto-nasale du bandeau orbito-naso-frontal lorsqu'en contact avec celui-ci. Les éléments rapportés latéraux peuvent être situés sur le support de manière à ce que les deux surfaces d'appui latérales se trouvent sensiblement au niveau des régions latérales du bandeau orbito-naso-frontal, lorsque en contact avec celui-ci.

Au moins un élément rapporté peut porter au moins une entretoise intercalée amovible permettant de modifier la longueur dudit élément rapporté. La taille totale d'entretoise(s) intercalée(s) sur ledit au moins un élément rapporté peut être corrélée de manière prédéterminée à la distance entre les sutures fronto-zygomatiques du patient.

Le gabarit peut présenter une symétrie autour d'un plan perpendiculaire au support, notamment l'élément rapporté central. Dans ce cas, la longueur d'au moins deux éléments rapportés, notamment latéraux, en particulier la taille totale d'entretoises intercalées sur ces éléments, peut être identique.

Il est à noter que le gabarit peut ne pas présenter de symétrie autour d'un plan horizontal traversant longitudinalement le support, l'appui central étant par exemple incliné d'un angle non nul, par exemple d'un angle d'environ 10° par rapport à un axe vertical.

Au moins un élément rapporté, notamment central, peut être de longueur fixe, non ajustable, étant par exemple dépourvu d'entretoise. En variante, deux éléments latéraux, disposés de part et d'autre d'un élément central, peuvent être de longueur fixe, l'élément central étant de longueur ajustable.

Selon un autre mode de réalisation, les éléments latéraux et l'élément central peuvent être de longueur fixe, la distance entre les éléments latéraux et centraux pouvant être modifiée, par exemple par l'intermédiaire d'entretoises. Dans ce dernier cas, on ajuste la longueur du support. Dans un autre cas particulier, on peut ajuster la longueur d'un ou de plusieurs éléments rapportés et la longueur du support, c'est-à-dire la distance entre au moins deux éléments en saillie, rapportés ou non, par exemple à l'aide d'entretoises. Le nombre de surfaces d'appui du gabarit peut être supérieur à trois, étant par exemple égal à cinq, ce qui peut permettre d'ajuster la courbure du bandeau orbito-naso-frontal, en plus de son angle d'ouverture.

Le gabarit selon l'invention peut comporter au moins un élément de guidage adapté pour replacer le bandeau orbito-naso-frontal sur le patient en respectant un angle naso-frontal physiologique. Ledit au moins un élément de guidage peut être choisi dans la liste constituée par : au moins deux éléments de guidage latéraux, notamment solidaires des éléments rapportés latéraux, et au moins un élément de guidage nasal, notamment solidaire d'un élément rapporté central.

Dans ce cas, le gabarit remplit en outre la fonction de guider le chirurgien dans le repositionnement du bandeau sur le patient, après remodelage.

Le gabarit peut comporter au moins un logement permettant au moins partiellement la mise en place d'un système de fixation du bandeau orbito-naso-frontal, le système de fixation pouvant consister en un fil, notamment métallique, par exemple en acier, et le logement étant choisi dans le groupe constitué par une échancrure, une encoche et un trou, notamment un trou oblong.

L'invention porte également, en combinaison avec ce qui précède, sur un ensemble de remodelage d'un bandeau orbito-naso-frontal d'un patient, comportant au moins un gabarit tel que défini plus haut et au moins une entretoise, de préférence un jeu d'entretoises, et des broches ou vis, pour permettre de modifier le positionnement de surfaces d'appui en fonction de la distance entre les sutures fronto-zygomatiques du patient. L'ensemble selon l'invention peut encore comporter un emballage contenant le gabarit et ladite au moins une entretoise et des vis et/ou broches, des clés hexagonales et/ou des tournevis, notamment dans des conditions stériles.

L'invention concerne également un procédé de fabrication d'un gabarit selon l'invention, comprenant les étapes suivantes :
- modéliser en numérique la forme d'un bandeau orbito-naso-frontal en se basant sur une distance entre les sutures fronto-zygomatiques prédéterminée, de préférence correspondant à un angle d'ouverture optimal de bandeau orbito-naso-frontal,
- déterminer en numérique le positionnement d'au moins trois surfaces d'appui à partir de cette modélisation,
- réaliser un gabarit physique avec un support et des éléments en saillie ou rapportés définissant lesdites surfaces d'appui.

Le procédé de fabrication peut encore comporter l'étape consistant à prévoir des vis, des broches, des clés hexagonales et/ou des tournevis pour maintenir les pièces ensemble.

L'invention concerne également, en combinaison avec ce qui précède, un procédé d'ajustement à un patient d'un gabarit selon l'invention, avec aucune ou au moins une entretoise, comportant les étapes suivantes :
- mesurer la distance entre les sutures fronto-zygomatiques du patient, de préférence en préopératoire,
- se rapporter à une droite de corrélation prédéfinie entre la taille totale d'entretoise et la distance entre les sutures fronto-zygomatiques, pour déterminer, à l'aide de cette droite, la taille totale d'entretoise à intercaler, pour ledit patient, sur le ou les éléments rapportés, notamment sur les éléments rapportés latéraux, respectivement entre les éléments rapportés, notamment entre l'élément central et un ou chaque élément latéral,
- intercaler et fixer aucune ou au moins une entretoise sur au moins un, notamment sur au moins deux éléments rapportés, respectivement entre des ou les éléments rapportés, pour obtenir la taille totale d'entretoise ainsi déterminée, qui peut être de 0 mm.

L'invention porte encore, en combinaison avec ce qui précède, sur un procédé de remodelage d'un bandeau orbito-naso-frontal d'un patient comportant les étapes suivantes :
- découper le bandeau orbito-naso-frontal déformé d'un patient,
- remodeler le bandeau orbito-naso-frontal en appuyant sur celui-ci un gabarit tel que défini plus haut, de manière à faire coïncider les surfaces d'appui aux zones correspondantes du bandeau orbito-naso-frontal,
- replacer le bandeau orbito-naso-frontal ainsi remodelé sur le patient.

Le procédé peut encore comporter l'étape consistant à ajuster la longueur d'au moins un élément rapporté pour correspondre à la distance mesurée sur le patient entre les sutures fronto-zygomatiques.

Grâce à l'invention, le chirurgien dispose d'un gabarit permettant de le guider vers un remodelage idéal du bandeau orbito-naso-frontal chez les patients atteints d'une craniosténose touchant une ou des sutures antérieures.

Le gabarit est personnalisable à chaque patient, étant adaptable à la distance entre les sutures fronto-zygomatiques mesurée en scanner préopératoire chez chaque patient.

L'invention pourra être mieux comprise à la lecture de la description détaillée qui va suivre, d'exemples de mise en œuvre non limitatifs de celle-ci et à l'examen du dessin annexé, sur lequel :
- la figure 1 est une vue schématique et en perspective d'un exemple de gabarit conforme à l'invention,
- la figure 2 est une vue schématique et en perspective d'un ensemble comportant le gabarit de la figure 1 et deux entretoises,
- la figure 3 est une photographie illustrant l'utilisation du gabarit des figures 1 et 2 pour remodeler un bandeau orbito-naso-frontal d'un patient,
- la figure 4 représente de manière schématique et en perspective un autre exemple de gabarit conforme à l'invention,
- la figure 5 est une coupe scannographique, en reconstruction coronale d'un crâne d'un patient afin de mesurer la distance entre les sutures fronto-zygomatiques,
- la figure 6 est une vue schématique en perspective d'un crâne vu de face sur lequel on a positionné un repère,
- la figure 7 est une vue schématique en perspective d'un crâne vu de côté, sur lequel on a positionné le repère de la figure 6,
- la figure 8 illustre une droite de corrélation entre la taille de l'entretoise totale et la distance entre les sutures fronto-zygomatiques,
- la figure 9 illustre un exemple de table de réglage illustrant la corrélation entre la taille de l'entretoise totale et la distance entre les sutures fronto-zygomatiques,
- la figure 10 illustre de manière schématique et en perspective la modélisation d'un bandeau orbito-naso-frontal pour la réalisation du gabarit selon l'invention,
- la figure 11 est une vue schématique et en perspective d'un autre exemple de réalisation du gabarit selon l'invention, et
- la figure 12 est une vue schématique et en perspective d'un autre exemple de réalisation du gabarit selon l'invention.

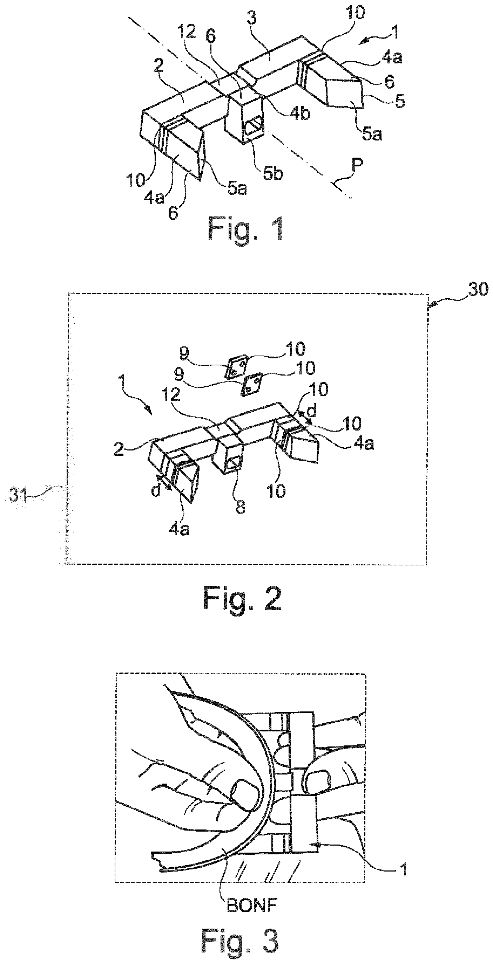

On a représenté sur la figure 1 un gabarit 1 chirurgical de remodelage du bandeau orbito-naso-frontal d'un patient souffrant de craniosténose antérieure. Comme visible, le gabarit 1 comporte un support 2 sous forme d'une barre 3, et des éléments en saillie, dans cet exemple des éléments rapportés 6 sous forme de branches 4. Les extrémités libres des éléments rapportés 6 ou branches 4 définissent des surfaces d'appui 5, planes dans cet exemple, destinées à être mises en contact avec le bandeau orbito-naso-frontal BONF prélevé sur le patient de manière à en remodeler la forme en per opératoire, comme visible sur la figure 3. Le positionnement relatif des surfaces d'appui 5 est réalisé, selon l'invention, en fonction de la taille du crâne définie par exemple par la DFZ, distance entre les sutures fronto-zygomatiques du patient concerné par l'opération.

Comme visible sur la figure 1, la barre 3 est rectiligne et les branches 4, également rectilignes, disposées perpendiculairement à la barre 3, sont sensiblement parallèles entre elles. Le gabarit 1 présente une symétrie autour d'un plan vertical passant par un axe P, horizontal. Le nombre de branches 4 est égal à trois dans cet exemple, à savoir deux branches latérales 4a et une branche centrale 4b, définissant trois surfaces d'appui 5, à savoir des surfaces d'appui 5a latérales et une surface d'appui 5 centrale 5b. La surface d'appui centrale 5b, traversée en son milieu par l'axe P, forme, dans l'exemple illustré, un angle non nul avec un plan vertical, tandis que les surfaces d'appui latérales 5a forment des angles non nuls avec l'axe P, dans un plan horizontal, comme visible et avec la verticale. La surface d'appui centrale 5b est, dans cet exemple, traversée par une ouverture 8 dont le rôle est de former un logement pour permettre de passer un système de fixation du bandeau BONF, tel qu'un fil métallique, notamment un fil d'acier. Le gabarit 1 comporte également dans cet exemple un logement formé par une encoche 12 réalisée sur le support 2 au niveau de la branche centrale 4b, l'encoche 12 étant prévue pour pouvoir mettre en place le système de fixation du bandeau BONF pour garder sa forme.

L'angle entre la surface d'appui centrale 5b et chacune des surfaces d'appui 5a latérales, différent de 90°, est déterminé par la forme d'un bandeau idéal, comme cela sera expliqué ci-après Dans cet exemple, les branches latérales 4a ont une longueur ajustable, dans cet exemple au moyen d'entretoises 10 que l'on peut intercaler, de manière amovible, et qui permettent de modifier la longueur de la branche latérale 4a concernée.

Pour remodeler de manière symétrique le bandeau orbito-naso-frontal, la longueur des branches latérales 4 est avantageusement identique. Ainsi, on dispose des entretoises 10 de manière à obtenir une taille totale d'entretoise qui soit identique entre les deux branches latérales 4a.

Par « taille totale d'entretoise », on entend la largeur totale de la ou des entretoises 10 intercalées dans la branche, les largeurs de deux entretoises adjacentes s'additionnant.

Le gabarit 1 peut être entièrement ou partiellement réalisé, notamment par usinage, en métal, notamment en acier tel que l'Inox 316L passivé. Le ou les matériau(x) utilisé(s) pour réaliser le gabarit doi(ven)t être stérilisable(s).

On a représenté sur la figure 2 un ensemble 30 de remodelage d'un bandeau BONF, comprenant le gabarit de la figure 1, représenté avec une taille totale d'entretoise d supérieure à celle de la figure 1. L'ensemble 30 comporte en outre au moins une entretoise 10, dont la longueur peut être nulle, notamment un jeu d'entretoises, au nombre de deux dans cet exemple, à disposer ou non sur le gabarit 1, en fonction de la distance DFZ du patient. L'ensemble 30 comporte également un emballage 31 représenté en pointillés sur la figure, pour contenir, notamment dans des conditions stériles, gabarit, entretoise(s), vis et/ou broches, clés hexagonales et tournevis.

Dans l'exemple illustré, pour obtenir la taille totale d'entretoise d souhaitée, on dispose une pluralité d'entretoises 10 de largeurs différentes. Les entretoises 10 sont percées de trous 9, au nombre de deux dans cet exemple, permettant le passage de broches présentes dans les branches latérales 4a, non visibles sur les figures dans un souci de clarté du dessin, afin de solidariser l'entretoise 10 à la branche 4. Les broches peuvent par exemple consister en deux vis cylindriques hexagonales creuses, par exemple en acier inoxydable. On ne sort pas du cadre de l'invention si un autre système de fixation de l'entretoise 10 est prévu. On ne sort pas non plus du cadre de l'invention si aucune ou une seule entretoise de largeur *d* est introduite dans la branche 4 concernée.

Dans l'exemple illustré, la branche centrale 4b présente une longueur fixe, non ajustable et est ainsi dépourvue d'entretoise. Il peut bien entendu en être différemment sans sortir du cadre de l'invention.

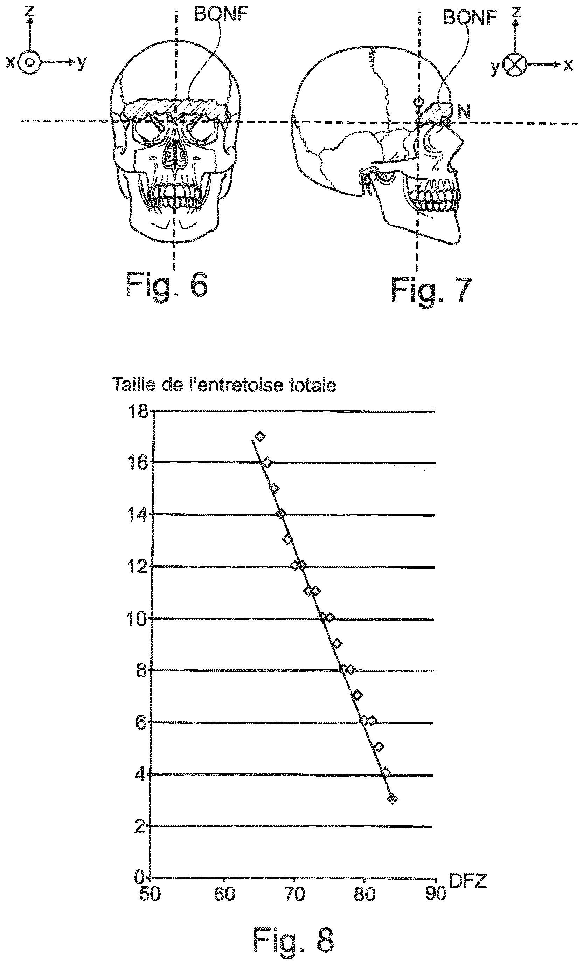

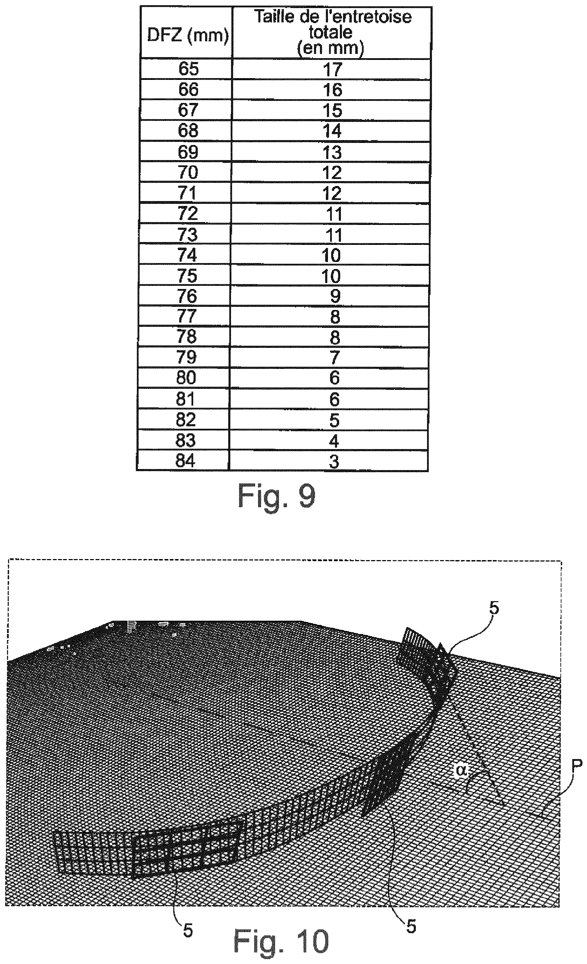

La taille totale *d* d'entretoise sur les branches latérales 4a est corrélée de manière prédéterminée à la distance entre les sutures fronto-zygomatiques DFZ du patient, selon la droite illustrée sur la figure 8 correspondant à la table de réglage illustrée sur la figure 9, réalisée par l'inventeur à partir d'une étude réalisée sur 123 patients ne présentant pas de craniosténose. La droite de la figure 8 et le tableau de la figure 9 sont liés à la taille de l'élément 6. On peut modifier ces données et la taille de l'élément 6 sans sortir du cadre de l'invention. La taille d=0 mm (pas d'entretoises) reste dans le cadre de l'invention.

Lors d'une opération chirurgicale, le chirurgien découpe le bandeau orbito-naso-frontal BONF déformé d'un patient, puis, comme illustré sur la figure 3, il remodèle le bandeau en appuyant sur celui-ci le gabarit 1 de manière à faire coïncider les surfaces d'appui aux zones correspondantes du bandeau BONF. En particulier, la surface d'appui centrale 5b, ou médiane, vient sensiblement au niveau de la suture fronto-nasale du bandeau et les deux surfaces d'appui latérales 5a, symétriques, sont positionnées sensiblement au niveau des régions latérales du bandeau.

A l'issue du remodelage, le bandeau BONF remodelé prend facilement appui sur les trois surfaces d'appui 5.

Enfin, selon ce procédé, on replace le bandeau ainsi remodelé sur le patient.

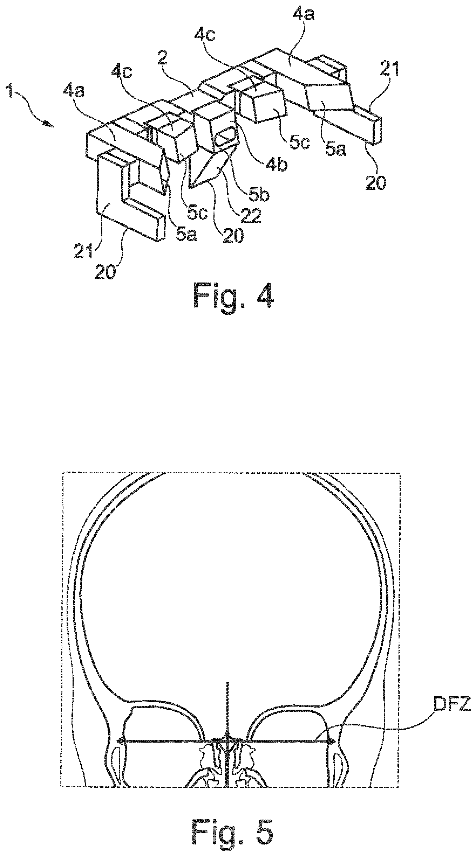

Dans un autre exemple illustré sur la figure 4, le gabarit 1 comporte cinq branches 4 définissant cinq surfaces d'appui 5, comme visible, deux branches 4c et surfaces d'appui 5c intermédiaires étant prévues entre les surfaces d'appui latérales 5a et la surface d'appui centrale 5b, permettant ainsi d'ajuster la courbure du bandeau BONF en plus de l'angle d'ouverture de celui-ci.

Egalement sur cette figure 4, le gabarit 1 comporte au moins un élément de guidage 20 adapté pour guider le chirurgien dans le repositionnement du bandeau BONF sur le patient, après remodelage, en respectant un angle naso-frontal physiologique. Les éléments de guidage 20 sont au nombre de trois dans cet exemple. Ils comprennent deux éléments de guidage latéraux 21, solidaires des branches latérales 4a, disposés latéralement à l'extérieur de celles-ci. Sur cette figure, ils se présentent sous forme d'équerres venant en contrebas et en avancée, latéralement, autour du gabarit 1, de manière à pouvoir se positionner latéralement autour du massif facial du patient.

Par ailleurs, un élément de guidage 20 consistant en un élément de guidage nasal 22, solidaire du support 2, notamment de la branche centrale 4b, est rapporté sous celle-ci et forme un angle avec un axe vertical de manière à venir épouser sensiblement l'orientation du nez du patient lors du repositionnement.

Comme évoqué plus haut, l'invention se base sur le constat, fait par l'inventeur lors de l'étude, qu'il existe une corrélation entre la distance entre les sutures fronto-zygomatiques DFZ et la forme du bandeau, notamment l'angle d'ouverture de celui-ci. Ainsi, à chaque distance entre les sutures fronto-zygomatiques DFZ correspond un groupe de valeurs statistiquement significatives permettant de caractériser le bandeau orbito-naso-frontal « idéal ».

On a illustré sur la figure 5 la distance entre les sutures fronto-zygomatiques DFZ telle qu'on peut la mesurer, notamment en préopératoire, sur scanner.

On a représenté sur les figures 6 et 7 le repère utilisé pour les mesures et pour l'étude consistant en un axe X horizontal traversant d'avant en arrière le crâne, et passant par le point N, appelé le nasion. Un axe Y, également horizontal, perpendiculaire à l'axe X, traverse le crâne latéralement et permet la mesure sur cet axe ou parallèlement à cet axe, de la distance DFZ. Enfin, un axe Z, vertical, passe par le point O centre du repère OXYZ correspondant au plan du sommet de la Crista Galli.

A l'aide de ce repère, de mesures effectuées sur de nombreux patients et de l'hypothèse que le bandeau BONF idéal est symétrique par rapport à un plan vertical centré, l'inventeur a pu modéliser la forme d'un bandeau BONF idéal, comme illustré sur la figure 10. Ce bandeau, d'une hauteur de 10 mm dans cet exemple, avec la coordonnée en Z comprise entre 10 et 20 mm, permet de modéliser également les trois surfaces d'appui 5, également visibles sur cette figure, notamment leur écartement, l'angle α fixe qu'elles forment avec l'axe P. Il est à noter que l'axe P est parallèle à l'axe X et que l'angle α est dans un plan horizontal contenant l'axe P.

Sur les figures 6 et 7, on a également représenté schématiquement le bandeau BONF, incluant le toit des orbites et une partie de l'os nasal.

La modélisation des surfaces d'appui 5 permet alors de construire le gabarit 1. Pour la construction des différentes positions des surfaces d'appui latérales, on utilise la table de réglage de la figure 9 et/ou la droite de corrélation de la figure 8. En effet, la taille totale d'entretoise nécessaire y est visible en fonction de la distance DFZ mesurée, par exemple par scanner en préopératoire, sur le patient à opérer.

Il est à noter que plus la DFZ est grande, plus la taille totale d'entretoise à intercaler est faible, c'est-à-dire plus la longueur des branches latérales est courte mais l'angle d'ouverture du bandeau BONF reste sensiblement constant car le point de contact sur le bandeau BONF se déplace. La possibilité de modifier les longueurs des éléments rapportés latéraux revient à la possibilité de modifier l'angle d'ouverture du bandeau à remodeler pour s'adapter à la DFZ d'un patient en particulier. Les tailles d'entretoises indiquées dans la table de réglage de la figure 9 permettent d'obtenir un angle d'ouverture optimal du bandeau BONF quelle que soit la valeur de la DFZ. Il est encore possible de ne pas avoir à intercaler d'entretoises pour obtenir la bonne longueur d'élément rapporté.

Lorsqu'un patient va être opéré, on mesure sur un scanner préopératoire la DFZ sur ce patient. On détermine alors, à l'aide de la table de réglage de la figure 9 ou la droite de corrélation de la figure 8, la taille totale d'entretoise nécessaire, et on ajuste la longueur des branches latérales 4 du gabarit 1 de manière à obtenir sur chacune d'elles la taille totale d'entretoise. Le gabarit 1 est alors prêt, après stérilisation, pour remodeler le bandeau orbito-naso-frontal qui sera prélevé sur le patient.

Lors de l'opération chirurgicale, une fois le bandeau réalisé, on le remodèle à l'aide du gabarit puis on le replace sur le patient.

Le gabarit est réutilisable pour une opération ultérieure, après stérilisation.

L'invention n'est pas limitée aux exemples qui viennent d'être décrits.

En particulier, l'ajustement de la longueur des éléments rapportés 6 ou branches 4 peut être réalisé non pas à l'aide d'entretoises mais à l'aide d'autres techniques d'ajustement incluant des tiges télescopiques, des tiges à crans traversant le support ou autres.

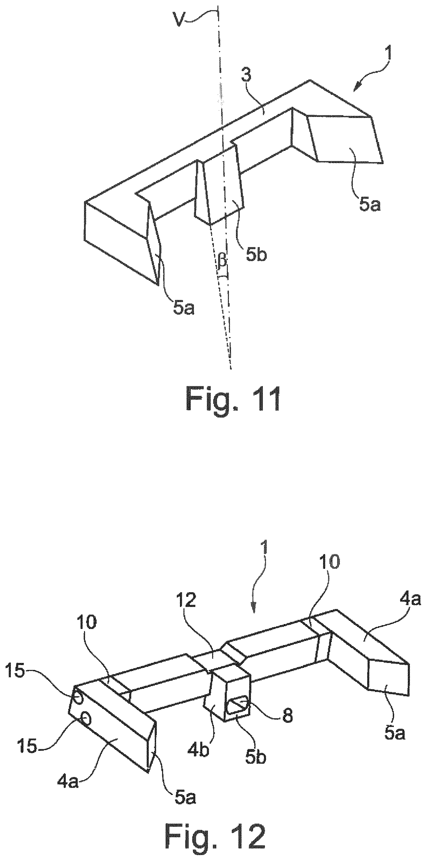

On a représenté sur la figure 11 un autre exemple de gabarit 1. Dans cet exemple, le nombre de surfaces d'appui est de trois, dont le positionnement change en fonction de la DFZ mesurée sur le patient, selon l'axe X ou Y. Ce gabarit 1 présente une symétrie par rapport à un plan vertical. La surface d'appui centrale 5b forme une surface plane présentant un angle β non nul avec un axe vertical V, angle pouvant par exemple être égal à 10°. Les surfaces latérales 5a présentent des angles non nuls avec la verticale et le plan de symétrie vertical.

On a représenté sur la figure 12 un autre exemple de gabarit 1. Dans cet exemple, l'entretoise 10, en l'espèce les entretoises 10, sont disposées sur la barre 3, à proximité des branches latérales 4a, afin d'augmenter la longueur de la barre 3, c'est-à-dire la distance relative entre les branches 4, notamment entre la branche centrale 4b et chacune des branches latérales 4a. Dans cet exemple, les longueurs des branches 4a et 4b sont fixes, étant non ajustables en longueur. On obtient ainsi un déplacement latéral des surfaces d'appui 5. Un système de broches 15, notamment de vis, au nombre de deux par côté, visible sur la figure 12, est prévu pour la mise en place ou retrait des entretoises 10.

Les surfaces d'appui du gabarit peuvent présenter une forme incurvée, concave ou convexe et/ou un relief en surface. La forme et/ou la surface des surfaces d'appui peut être modifiée sans sortir du cadre de l'invention.

Au moins un ou les éléments en saillie formant les branches 4 peuvent être réalisés d'une seule pièce avec le support, par exemple par impression en trois dimensions ou par fraisage dans la masse.

La taille du crâne peut être mesurée par une autre valeur que la distance entre les sutures zygomatiques DFZ, sans sortir du cadre de l'invention.

L'expression modèle ou modélisation doit être comprise comme modèle (ou modélisation) numérique ou digital (sur ordinateur).

Dans toute la description, l'expression « comportant un » doit être comprise comme étant synonyme de l'expression « comprenant au moins un ».

## Revendications

1. Gabarit (1) chirurgical de remodelage de bandeau orbito-naso-frontal, comportant :
- un support (2), et
- au moins trois surfaces d'appui (5) destinées à être mises en contact avec un bandeau orbito-naso-frontal (BONF) réalisé sur un patient de manière à en remodeler la forme en per opératoire,
les surfaces d'appui étant définies par des éléments en saillie relativement au support (2), au moins un élément en saillie étant un élément rapporté (6) sur le support (2), au moins un élément rapporté (6) présentant une longueur ajustable et/ou la distance entre des éléments en saillie ou rapportés (6) étant modifiable, **caractérisé par le fait que** les surfaces d'appui (5) sont configurées de telle sorte que leur positionnement relativement au support est fonction de la taille du crâne mesurée en préopératoire.

2. Gabarit (1) selon la revendication 1, dans lequel le positionnement des surfaces d'appui (5) relativement au support est fonction de la taille du crâne mesurée par la distance entre les sutures fronto-zygomatiques (DFZ) dudit patient, mesurée dans un plan scannographique donné, en reconstruction coronale dans le plan du sommet de la crista galli, selon une mesure reproductible.

3. Gabarit (1) selon la revendication 1 ou 2, dans lequel au moins un élément rapporté (6) est configuré de telle sorte que le positionnement de la surface d'appui (5) qu'il définit peut être modulé entre au moins une première position et au moins une deuxième position différente de la première position, au moins un élément rapporté (6) présentant de préférence une longueur ajustable de manière à permettre à la surface d'appui (5) qu'il définit d'occuper lesdites au moins deux positions, la première position de la surface d'appui (5) étant notamment déterminée à partir d'une reconstitution en trois dimensions d'un bandeau orbito-naso-frontal de forme idéale ayant une distance entre les sutures fronto-zygomatiques (DFZ) qui est prédéterminée, notamment comprise entre 60 et 90 mm et au moins une deuxième position de la surface d'appui (5) étant de préférence déterminée à partir d'une reconstitution en trois dimensions d'un bandeau orbito-naso-frontal de forme idéale ayant une distance entre les sutures fronto-zygomatiques qui est prédéterminée, comprise entre 60 et 90 mm.

4. Gabarit (1) selon l'une quelconque des revendications précédentes, dans lequel au moins une surface d'appui (5) est plane.

5. Gabarit (1) selon l'une quelconque des revendications précédentes, dans lequel les éléments rapportés (6) comportent un élément rapporté central (4b) définissant une surface d'appui centrale (5b) et au moins deux éléments rapportés latéraux (4a), de part et d'autre de l'élément rapporté central (4b), définissant au moins deux surfaces d'appui latérales (5a).

6. Gabarit (1) selon l'une quelconque des revendications précédentes, dans lequel au moins un élément rapporté (6) porte au moins une entretoise (10) intercalée amovible permettant de modifier la longueur dudit élément rapporté (6), la taille totale (*d*) d'entretoise (10) intercalée sur ledit au moins un élément rapporté (6) étant de préférence corrélée de manière prédéterminée à la distance entre les sutures fronto-zygomatiques (DFZ) du patient.

7. Gabarit (1) selon l'une quelconque des revendications précédentes, présentant une symétrie autour d'un plan, vertical, perpendiculaire au support (2), la longueur d'au moins deux éléments rapportés (6), notamment latéraux, étant identique.

8. Gabarit (1) selon l'une quelconque des revendications précédentes, dans lequel au moins un élément rapporté (6) est de longueur fixe, non ajustable.

9. Gabarit selon l'une quelconque des revendications précédentes, dans lequel la distance entre les éléments en saillie ou rapportés (6) est modifiable, notamment par l'intermédiaire d'entretoises (10).

10. Gabarit (1) selon l'une quelconque des revendications précédentes, dans lequel le nombre de surfaces d'appui (5) est supérieur à trois, étant par exemple égal à cinq.

11. Gabarit (1) selon l'une quelconque des revendications précédentes, comportant au moins un élément de guidage (20) adapté pour replacer le bandeau orbito-naso-frontal sur le patient en respectant un angle naso-frontal physiologique, ledit au moins un élément de guidage (20) étant de préférence choisi dans la liste constituée par : au moins deux éléments de guidage latéraux (21), notamment solidaires d'éléments rapportés latéraux, et au moins un élément de guidage nasal (22), notamment solidaire d'un élément rapporté central.

12. Gabarit (1) selon l'une quelconque des revendications précédentes, comportant au moins un logement permettant au moins partiellement la mise en place d'un système de fixation du bandeau orbito-naso-frontal, le système de fixation consistant notamment en un fil métallique, et le logement étant choisi dans le groupe constitué par une échancrure, une encoche et un trou.

13. Ensemble (30) de remodelage d'un bandeau orbito-naso-frontal d'un patient, comportant au moins un gabarit (1) selon l'une quelconque des revendications 1 à 12 et au moins une entretoise (10), de préférence un jeu d'entretoises et de broches ou vis, pour permettre de modifier le positionnement de surfaces d'appui (5) du gabarit (1) en fonction de la distance entre les sutures fronto-zygomatiques (DFZ) du patient, l'ensemble comportant de préférence un emballage (31) contenant le gabarit (1) et ladite au moins une entretoise (10) et des vis et/ou broches, des clés hexagonales et/ou des tournevis, notamment dans des conditions stériles.

14. Procédé de fabrication d'un gabarit (1) selon l'une quelconque des revendications 1 à 12, comprenant les étapes suivantes :
- modéliser en numérique la forme d'un bandeau orbito-naso-frontal (BONF) en se basant sur une distance entre les sutures fronto-zygomatiques (DFZ) prédéterminée, de préférence correspondant à un angle d'ouverture optimal de bandeau orbito-naso-frontal,
- déterminer en numérique le positionnement de surfaces d'appui (5) à partir de cette modélisation,
- réaliser un gabarit (1) physique avec un support (2) et des éléments en saillie ou rapportés (6) définissant lesdites surfaces d'appui (5).

15. Procédé d'ajustement à un patient d'un gabarit (1) selon la revendication 6 et éventuellement l'une quelconque des revendications 1 à 12, avec au moins une entretoise (10), comportant les étapes suivantes :
- mesurer la distance entre les sutures fronto-zygomatiques (DFZ) du patient, de préférence en préopératoire,
- se rapporter à une droite de corrélation entre la taille totale d'entretoise et la distance entre les sutures fronto-zygomatiques (DFZ), pour déterminer, à l'aide de cette droite, la taille totale d'entretoise (*d*) à intercaler, pour ledit patient, sur le ou les éléments rapportés (6), notamment sur les éléments rapportés latéraux, respectivement entre les éléments rapportés,
- intercaler et fixer au moins une entretoise (10) sur au moins un, notamment au moins deux éléments rapportés (6), respectivement entre des ou les éléments rapportés, pour obtenir la taille totale d'entretoise (*d*) ainsi déterminée.

## Patentansprüche

1. Chirurgische Schablone (1) zur Remodellierung eines frontoorbitonasalen Bands, umfassend:
- einen Träger (2) und
- zumindest drei Auflageflächen (5), die dazu bestimmt sind, mit einem an einem Patienten ausgeführten frontoorbitonasalen Band (BONF) in Kontakt gebracht zu werden, um seine Form während eines operativen Eingriffs zu remodellieren,
wobei die Auflageflächen durch relativ zum Träger (2) vorstehende Elemente definiert sind, wobei zumindest ein vorstehendes Element ein am Träger (2) angebrachtes Element (6) ist, wobei zumindest ein angebrachtes Element (6) eine einstellbare Länge aufweist und/oder wobei der Abstand zwischen vorstehenden bzw. angebrachten Elementen (6) veränderbar ist, **dadurch gekennzeichnet, dass** die Auflageflächen (5) so gestaltet sind, dass ihre Positionierung relativ zum Träger von der präoperativ gemessenen Schädelgröße abhängig ist.

2. Schablone (1) nach Anspruch 1, wobei die Positionierung der Auflageflächen (5) relativ zum Träger abhängig ist von der Größe des Schädels, gemessen durch den Abstand zwischen den frontozygomatischen Nähten (DFZ) des Patienten, gemessen in einer gegebenen Scanebene, bei koronarer Rekonstruktion in der Ebene der Spitze der Crista galli, gemäß einer reproduzierbaren Messung.

3. Schablone (1) nach Anspruch 1 oder 2, wobei zumindest ein angebrachtes Element (6) so gestaltet ist, dass die Positionierung der von ihm definierten Auflagefläche (5) zwischen zumindest einer ersten Position und zumindest einer von der ersten Position verschiedenen zweiten Position modulierbar ist, wobei zumindest ein angebrachtes Element (6) vorzugsweise eine Länge aufweist, die so einstellbar ist, dass sie es der von ihm definierten Auflagefläche (5) ermöglicht, die zumindest zwei Positionen einzunehmen, wobei die erste Position der Auflagefläche (5) insbesondere anhand einer dreidimensionalen Rekonstruktion eines ideal geformten frontoorbitonasalen Bands mit einem vorbestimmten Abstand zwischen den frontozygomatische Nähten (DFZ), insbesondere im Bereich von 60 bis 90 mm, bestimmt wird und wobei zumindest eine zweite Position der Auflagefläche (5) vorzugsweise anhand einer dreidimensionalen Rekonstruktion eines ideal geformten frontoorbitonasalen Bands mit einem vorbestimmten Abstand zwischen den frontozygomatischen Nähten im Bereich von zwischen 60 und 90 mm bestimmt wird.

4. Schablone (1) nach einem der vorangehenden Ansprüche, wobei zumindest eine Auflagefläche (5) eben ist.

5. Schablone (1) nach einem der vorangehenden Ansprüche, wobei die angebrachten Elemente (6) ein mittleres angebrachtes Element (4b), das eine mittlere Auflagefläche (5b) definiert, und zumindest zwei seitliche angebrachte Elemente (4a) auf jeder Seite des mittleren angebrachten Elements (4b), die zumindest zwei seitliche Auflageflächen (5a) definieren, umfassen.

6. Schablone (1) nach einem der vorangehenden Ansprüche, wobei zumindest ein angebrachtes Element (6) zumindest einen herausnehmbar eingefügten Abstandhalter (10) trägt, der es ermöglicht, die Länge des angebrachten Elements (6) zu ändern, wobei die Gesamtgröße (d) des am zumindest einen angebrachten Element (6) eingefügten Abstandhalters (10) vorzugsweise in vorbestimmter Weise mit dem Abstand zwischen den frontozygomatischen Nähten (DFZ) des Patienten korreliert.

7. Schablone (1) nach einem der vorangehenden Ansprüche, die eine Symmetrie um eine vertikale Ebene senkrecht zum Träger (2) aufweist, wobei die Länge von zumindest zwei angebrachten, insbesondere seitlichen Elementen (6) identisch ist.

8. Schablone (1) nach einem der vorangehenden Ansprüche, wobei zumindest ein angebrachtes Element (6) eine feste, nicht einstellbare Länge aufweist.

9. Schablone nach einem der vorangehenden Ansprüche, wobei der Abstand zwischen den vorstehenden bzw. angebrachten Elementen (6) veränderbar ist, insbesondere anhand von Abstandhaltern (10).

10. Schablone (1) nach einem der vorangehenden Ansprüche, wobei die Anzahl von Auflageflächen (5) größer als drei, beispielsweise gleich fünf, ist.

11. Schablone (1) nach einem der vorangehenden Ansprüche, umfassend zumindest ein Führungselement (20), das dazu ausgelegt ist, das frontoorbitonasale Band unter Einhaltung eines physiologischen frontonasalen Winkels am Patienten anzuordnen, wobei das zumindest eine Führungselement (20) vorzugsweise ausgewählt ist aus der Liste, umfassend: zumindest zwei seitlichen Führungselementen (21), die insbesondere fest mit seitlichen angebrachten Elemente verbunden sind, und zumindest einem nasalen Führungselement (22), das insbesondere fest mit einem mittleren angebrachten Element verbunden ist.

12. Schablone (1) nach einem der vorangehenden Ansprüche, umfassend zumindest eine Aufnahme, die zumindest teilweise die Anordnung eines Befestigungssystems des frontoorbitonasalen Bands ermöglicht, wobei das Befestigungssystem insbesondere aus einem Metalldraht besteht und wobei die Aufnahme ausgewählt ist aus der Gruppe, umfassend eine Einkerbung, eine Aussparung und ein Loch.

13. Baugruppe (30) zur Remodellierung eines frontoorbitonasalen Bands eines Patienten, umfassend zumindest eine Schablone (1) nach einem der Ansprüche 1 bis 12 und zumindest einen Abstandhalter (10), vorzugsweise einen Satz Abstandhalter, und Stifte oder Schrauben, um zu ermöglichen, die Positionierung der Auflageflächen (5) der Schablone (1) in Abhängigkeit vom Abstand zwischen den frontozygomatischen Nähten (DFZ) des Patienten zu modifizieren, wobei die Baugruppe vorzugsweise eine Verpackung (31) umfasst, die die Schablone (1) und den zumindest einen Abstandhalter (10) und Schrauben und/oder Stifte, Sechskantschlüssel und/oder Schraubendreher enthält, insbesondere unter sterilen Bedingungen.

14. Verfahren zur Herstellung einer Schablone (1) nach einem der Ansprüche 1 bis 12, umfassend die folgenden Schritte:
- numerisches Modellieren der Form eines frontoorbitonasalen Bands (BONF) auf der Grundlage eines vorbestimmten Abstands zwischen den frontozygomatischen Nähten (DFZ), der vorzugsweise einem optimalen Öffnungswinkel des frontoorbitonasalen Bands entspricht,
- numerisches Bestimmen der Positionierung der Auflageflächen (5) anhand dieser Modellierung,
- Ausführen einer physischen Schablone (1) mit einem Träger (2) und vorstehenden bzw. angebrachten Elementen (6), die die Auflageflächen (5) definieren.

15. Verfahren zum Anpassen einer Schablone (1) nach Anspruch 6 und gegebenenfalls einem der Ansprüche 1 bis 12 mit zumindest einem Abstandhalter (10) an einen Patienten, umfassend die folgenden Schritte:
- Messen des Abstands zwischen den frontozygomatischen Nähten (DFZ) des Patienten, vorzugsweise präoperativ,
- Bezugnehmen auf eine Korrelationslinie zwischen der Abstandhaltergesamtgröße und dem Abstand zwischen den frontozygomatischen Nähten (DFZ), um anhand dieser Linie die Gesamtgröße des Abstandhalters (d) zu bestimmen, der für den Patienten an dem bzw. den angebrachte(n) Elemente(n) (6), insbesondere an den seitlichen angebrachten Elementen bzw. zwischen den angebrachten Elementen, einzusetzen ist,
- Einsetzen und Befestigen zumindest eines Abstandhalters (10) an zumindest einem, insbesondere zumindest zwei angebrachten Elemente (6), jeweils zwischen dem bzw. den angebrachte(n) Elemente(n), um die so bestimmte Abstandhaltergesamtgröße (d) zu erhalten.

## Claims

1. Surgical template (1) for remodelling of an orbito-naso-frontal bandeau, comprising:
- a support (2), and
- at least three bearing surfaces (5) intended to be placed in contact with an orbito-naso-frontal bandeau (ONFB) taken from a patient, so as to remodel its shape intraoperatively,
the bearing surfaces being defined by elements protruding relative to the support (2), at least one protruding element being an element (6) attached to the support (2), at least one attached element (6) having an adjustable length and/or the distance between protruding or attached elements (6) being modifiable, **characterized in that** the bearing surfaces (5) are configured such that their positioning relative to the support depends on the size of the cranium measured pre-operatively.

2. Template (1) according to Claim 1, in which the positioning of the bearing surfaces (5) relative to the support depends on the size of the cranium measured by the distance between the fronto-zygomatic sutures (FZD) of said patient, measured in a given CT plane, with coronal reconstruction in the plane of the apex of the crista galli, by a reproducible measurement.

3. Template (1) according to Claim 1 or 2, in which at least one attached element (6) is configured in such a way that the positioning of the bearing surface (5) that it defines can be adapted between at least one first position and at least one second position different from the first position, at least one attached element (6) preferably having an adjustable length in such a way as to allow the bearing surface (5) that it defines to occupy said at least two positions, the first position of the bearing surface (5) being determined in particular from a three-dimensional reconstruction of an orbito-naso-frontal bandeau of ideal shape having a distance between the fronto-zygomatic sutures (FZD) which is predetermined, in particular of between 60 and 90 mm, and at least one second position of the bearing surface (5) preferably being determined from a three-dimensional reconstruction of an orbito-naso-frontal bandeau of ideal shape having a distance between the fronto-zygomatic sutures which is predetermined, of between 60 and 90 mm.

4. Template (1) according to any one of the preceding claims, in which at least one bearing surface (5) is plane.

5. Template (1) according to any one of the preceding claims, in which the attached elements (6) comprise a central attached element (4b) defining a central bearing surface (5b), and at least two lateral attached elements (4a), on either side of the central attached element (4b), defining at least two lateral bearing surfaces (5a).

6. Template (1) according to any one of the preceding claims, in which at least one attached element (6) carries at least one spacer (10) inserted removably and allowing the length of said attached element (6) to be modified, the total size (d) of inserted spacer (10) on said at least one attached element (6) preferably being correlated in a predetermined manner to the distance between the fronto-zygomatic sutures (FZD) of the patient.

7. Template (1) according to any one of the preceding claims, having a symmetry about a vertical plane perpendicular to the support (2), the length of at least two attached elements (6), in particular lateral ones, being identical.

8. Template (1) according to any one of the preceding claims, in which at least one attached element (6) is of fixed, non-adjustable length.

9. Template according to any one of the preceding claims, in which the distance between the protruding or attached elements (6) is modifiable, in particular by way of spacers (10).

10. Template (1) according to any one of the preceding claims, in which the number of bearing surfaces (5) is greater than three, for example equal to five.

11. Template (1) according to any one of the preceding claims, comprising at least one guide element (20) for repositioning the orbito-naso-frontal bandeau on the patient according to a physiological naso-frontal angle, said at least one guide element (20) preferably being chosen from the list composed of: at least two lateral guide elements (21), in particular rigidly connected to lateral attached elements, and at least one nasal guide element (22), in particular rigidly connected to a central attached element.

12. Template (1) according to any one of the preceding claims, comprising at least one recess permitting at least partially the placement of a system for fixing the orbito-naso-frontal bandeau, the fixing system consisting in particular of a metal wire, and the recess being chosen from the group composed of an indentation, a notch and a hole.

13. Assembly (30) for remodelling an orbito-naso-frontal bandeau of a patient, comprising at least one template (1) according to any one of Claims 1 to 12 and at least one spacer (10), preferably a set of spacers and pins or screws, in order to be able to modify the positioning of bearing surfaces (5) of the template (1) depending on the distance between the fronto-zygomatic sutures (FZD) of the patient, the assembly preferably comprising a package (31) containing the template (1) and said at least one spacer (10) and screws and/or pins, hex keys and/or screwdrivers, in particular under sterile conditions.

14. Method for producing a template (1) according to any one of Claims 1 to 12, comprising the following steps:
- digital modelling of the shape of an orbito-naso-frontal bandeau (ONFB) based on a predetermined distance between the fronto-zygomatic sutures (FZD), preferably corresponding to an optimal angle of opening of the orbito-naso-frontal bandeau,
- digital determination of the positioning of bearing surfaces (5) based on this modelling,
- production of a physical template (1) with a support (2) and with protruding or attached elements (6) defining said bearing surfaces (5).

15. Method for adapting to a patient a template (1) according to Claim 6 and if appropriate according to any one of Claims 1 to 12, with at least one spacer (10), said method comprising the following steps:
- measuring the distance between the fronto-zygomatic sutures (FZD) of the patient, preferably pre-operatively,
- referring to a correlation straight line between the total spacer size and the distance between the fronto-zygomatic sutures (FZD) in order to determine, with the aid of this straight line, the total spacer size (d) to be inserted, for said patient, on the one or more attached elements (6), in particular on the lateral attached elements, respectively between the attached elements,
- inserting and fixing at least one spacer (10) on at least one attached element (6), in particular at least two attached elements (6), respectively between the one or more attached elements, in order to obtain the total spacer size (d) thus determined.
